# EUROPEAN PATENT SPECIFICATION

(11) **EP 3 064 553 B1**
(45) Date of publication and mention of the grant of the patent: **24.03.2021**
(21) Application number: 14857699.4
(22) Date of filing: 28.10.2014
(51) Int. Cl.: C09C 3/06, C09C 1/00, C09C 1/24

(54) **IRON OXIDE BASED BLACK GLOSSY PIGMENT AND METHOD FOR PREPARING SAME**
EISENOXIDBASIERTES GLÄNZENDES SCHWARZES PIGMENT UND VERFAHREN ZUR HERSTELLUNG DAVON
PIGMENT BRILLANT NOIR À BASE D'OXYDE DE FER ET SON PROCÉDÉ DE PRÉPARATION

(30) Priority: 28.10.2013 KR 20130128826
(43) Date of publication of application: 07.09.2016
(73) Proprietor: Cqv Co., Ltd., Jincheon-gun, Chungcheongbuk-do 365-802 (KR)
(72) Inventor: HEO, Dong-Min, Pyeongtaek-si Gyeonggi-do 451-806 (KR); JEONG, Jae-Il, Cheongju-si Chungcheongbuk-do 361-713 (KR); KANG, Kwang-Choong, Cheongju-si Chungcheongbuk-do 360-826 (KR); CHOI, Byung-Ki, Cheongju-si Chungcheongbuk-do 363-781 (KR); LIM, Kwang-Soo, Jincheon-gun Chungcheongbuk-do 365-802 (KR); CHANG, Kil-Wan, Cheongju-si Chungcheongbuk-do 361-951 (KR); JANG, Gun-Eik, Cheongju-si Chungcheongbuk-do 360-770 (KR)
(74) Representative: Wynne-Jones IP Limited
(86) International application number: PCT/KR2014/010211
(87) International publication number: WO 2015/065026

(56) References cited:
- EP-A2- 2 511 348
- KR-A- 20100 004 252
- KR-B1- 100 736 859
- KR-B1- 100 736 859
- KR-B1- 101 135 360
- US-A- 4 090 888
- US-A1- 2012 237 577
- US-B2- 7 303 622
- XIAOJUAN LIANG ET AL: "Research of mica/FeOpearlescent pigment by co-precipitation", GLASS PHYSICS AND CHEMISTRY, KLUWER ACADEMIC PUBLISHERS-PLENUM PUBLISHERS, NE, vol. 37, no. 3, 29 June 2011 (2011-06-29), pages 330-342, XP019920693, ISSN: 1608-313X, DOI: 10.1134/S1087659611030084

## Description

### [Technical Field]

The present invention relates to a technology for preparing an iron oxide-based black glossy pigment, and more specifically, to an iron oxide-based black glossy pigment capable of securing stability of a reaction process and reducing process cost, and a method for preparing the same.

### [Background Art]

Pigments have been used to have an aesthetic effect in various fields. In order to express various colors, various metals or metal oxides such as titanium dioxide, iron oxide, silicon dioxide, etc., have been used alone or mixed with each other.

Among them, the existing black pigment is mainly developed by processing a cobalt compound, but since the cobalt is harmful, applications of the black pigment are limited.

In addition, there are many cases that the pigment is mixed with resins according to applications. However, in this case, in order to form patterns on a surface on which the pigment is applied, the patterns are firstly formed by a paint mixed with the pigment through gravure printing, etc., and additional pain is applied thereonto, such that operation processes are complicated. In order to compensate supplement these disadvantages, an iron oxide-based black pigment having a strong magnetic effect has been developed.

The existing iron oxide-based black glossy pigment is prepared by reducing a surface of iron oxide (Fe₂O₃) under high temperature and high pressure or by sintering iron oxide in a reducing furnace. However, these methods have disadvantages in that it is difficult to secure stability in reaction processes and production cost is increased according to reaction conditions. In particular, the reducing furnace has a possibility of explosion due to hydrogen inflow.

Korean Patent No. 10-0238859 (Registered on Oct. 6, 1999) disclosed a magnetic iron oxide pigment and a manufacturing method thereof as a Patent Document relevant to the present invention.

Other relevant publications include KR100736859, US2012/237577, US7303622, and Liang, X. et al., Glass Physics and Chemistry, 2011, Vol. 37, No. 3, pp. 330-342. KR100736859 discloses a black glazed pigment for cosmetics, which shows excellent black-colored gloss without using any conventional black pigment, and is completely cleaned from the skin while not leaving any black after-color. US2012/237577 discloses a black effect pigment and a method of forming said pigment. US7303622 discloses a lustrous black interference pigment based on a substrate mixture. Liang, X *et al.* discloses research of mica/Fe₃O₄ pearlescent pigment by co-precipitation.

### [Disclosure]

### [Technical Problem]

It is an aspect of the present invention to provide a method for preparing an iron oxide-based black glossy pigment comprising iron oxide (Fe₃O₄) by using a harmless iron (Fe²⁺) metal salt as a starting material, instead of using harmful cobalt (Co) that is a main component of the existing black glossy pigment, etc.

In particular, it is another aspect of the present invention to provide a method for preparing an iron oxide-based black glossy pigment having a low reaction temperature without additionally performing a sintering process, as compared to the existing methods for preparing a soft magnetic iron oxide.

### [Technical Solution]

The present invention is set out in the appended claims. Any embodiments, aspects or examples of the present description/disclosure that do not fall within the scope of said claims are provided for illustrative purposes only and do not form part of the present invention.

In accordance with one aspect of the present invention, a method for preparing an Fe₃O₄ iron oxide-based black glossy pigment includes: preparing an iron sulfate dilution liquid containing iron sulfate (FeSO₄·7H₂O); forming a substrate suspension by adding flake substrates to D.I. water, followed by mixing, stirring and dispersing; coating the flake substrates by titrating the substrate suspension with an aqueous inorganic salt solution to hydrolyze the aqueous inorganic salt solution, and mixing the prepared iron sulfate dilution liquid therewith, to thereby coat an oxide layer on surfaces of the flake substrates, and after the coating of the flake substrates, dehydrating and washing the flake substrates coated with the oxide layer and a reaction residual liquid, and drying the dehydrated flake substrates at 50 °C to 60 °C, and screening the flake substrates, using mesh,
wherein the aqueous inorganic salt solution includes one selected from TiCl4, TiOCl2, TiOSO4, BaCl2, AlCl3, SnCl4, FeCl3, FeSO4, SiCl4, ZrOCl2, Na2O·SiO2·5H2O, MnCl2, MgCl2 and CoCl2, or one or more mixtures thereof, and
wherein in the coating of the flake substrates(S130), at a pH of 7 to 10, the substrate suspension is stirred and heated at 80 °C to 85 °C at 400 rpm to 450 rpm, and the iron sulfate dilution liquid is added dropwise to the substrate suspension at a rate of 200 mL/hr to 300 mL/hr.

### [Advantageous Effects]

According to a method for preparing an iron oxide-based black glossy pigment of the present invention, the iron oxide-based black glossy pigment including Fe₃O₄ may be formed by using Fe²⁺ metal salt as a starting material rather than mixing Fe²⁺ with Fe³⁺ salt to form a pigment including Fe₃O₄ according to the related art.

In addition, since the method of the present invention has a relatively low reaction temperature and does not require an additional sintering process, as compared to a method for reducing a surface of Fe₂O₃ and a method for sintering Fe₂O₃ under reduction conditions, both of which are the existing methods for preparing a soft magnetic iron oxide, stability of a reaction process may be secured, and the production cost may be reduced.

### [Description of Drawings]

FIG. 1 is a flow chart showing a method for preparing an iron oxide-based black glossy pigment according to an exemplary embodiment of the present invention.
FIG. 2 is a graph showing saturation magnetization of an iron oxide-based black pigment glossy pigment prepared according to Comparative Example.
FIG. 3 is a graph showing magnetic saturation of an iron oxide-based black pigment glossy pigment prepared according to Example of the present invention.
FIG. 4 is a scanning electron microscope (SEM) image of a surface of the iron oxide-based black pigment glossy pigment prepared according to Example of the present invention.

### [Best Mode]

Hereinafter, a method for preparing an iron oxide-based black glossy pigment using iron sulfate according to an exemplary embodiment of the present invention and an iron oxide-based black glossy pigment prepared by using the same are described.

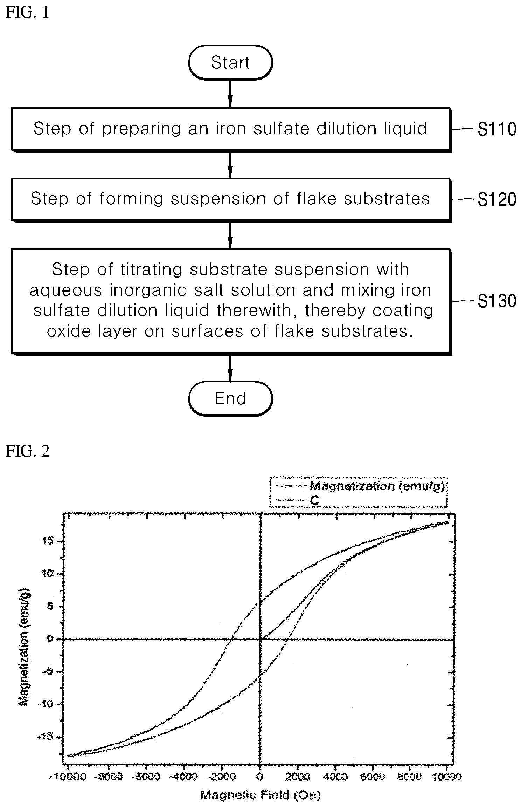

FIG. 1 is a flow chart showing a method for preparing an iron oxide-based black glossy pigment using iron sulfate according to an exemplary embodiment of the present invention.

Referring to FIG. 1, the method for preparing the iron oxide-based black glossy pigment using the iron sulfate according to an exemplary embodiment of the present invention includes: preparing an iron sulfate dilution liquid (S110); forming a substrate suspension by adding flake substrates to deionized water (D.I. water), followed by mixing, stirring and dispersing (S120); and coating the flake substrates by titrating the substrate suspension with an aqueous inorganic salt solution to hydrolyze the aqueous inorganic salt solution, and titrating the prepared iron sulfate dilution liquid therewith, to thereby coat an oxide layer on surfaces of the flake substrates through hydrothermal synthesis (S130).

The preparing of the iron sulfate dilution liquid (S110) includes: preparing a sulfuric acid dilution liquid by mixing 100 parts by weight of water with 1 to 6 parts by weight of sulfuric acid, preparing a first mixed solution containing iron sulfate and acid by mixing 100 parts by weight of the prepared sulfuric acid dilution liquid with 10 to 40 parts by weight of iron sulfate (FeSO₄·7H₂O), and preparing a second mixed solution by mixing and stirring 100 parts by weight of the first mixed solution with 3 to 10 parts by weight of at least one of KNO₃, NaNO₃, Ca(NO₃)₂ and Mg(NO₃)₂.

First, the sulfuric acid dilution liquid is prepared by mixing 100 parts by weight of water with 1 to 6 parts by weight of sulfuric acid.

Then, the first mixed solution containing iron sulfate and acid is prepared by mixing 100 parts by weight of the prepared sulfuric acid dilution liquid with 10 to 40 parts by weight of iron sulfate (FeSO₄·7H₂O).

Next, the second mixed solution is prepared by mixing 100 parts by weight of the first mixed solution with 3 to 10 parts by weight of at least one of KNO₃, NaNO₃, Ca(NO₃)₂ and Mg(NO₃)₂, followed by stirring for 30 minutes to 50 minutes.

In the forming of the substrate suspension (S120), the flake substrate may include at least one of synthetic mica, natural mica, glass, plate-shaped iron oxide, plate-shaped alumina and plate-shaped silica, talc, lead carbonate, and plate-shaped bismuth oxychloride (BiOCI).

In the forming of the substrate suspension, 100 parts by weight of the flake substrates are mixed with 900 to 1200 parts by weight of D.I. water, followed by heating and stirring at 70 °C to 80 °C at 400 rpm to 450 rpm.

In the coating of the flake substrates (S130), the aqueous inorganic salt solution may include one selected from TiCl₄, TiOCl₂, TiOSO₄, BaCl₂, AlCl₃, SnCl₄, FeCl₃, FeSO₄, SiCl₄, ZrOCl₂, Na₂O·SiO₂·5H₂O, MnCl₂, MgCl₂ and CoCl₂, or one or more mixtures thereof.

The substrate suspension formed in the forming of the substrate suspension (S120) is stirred and heated at 70°C to 80°C at 400 rpm to 450 rpm, and pH is maintained to 1.5 to 2.5. Then, 8 to 12 parts by weight of the aqueous inorganic salt solution is added dropwise to 100 parts by weight of the substrate suspension to hydrolyze the aqueous inorganic salt solution.

Then, a temperature of iron sulfate dilution liquid is raised to 80°C to 85°C, and pH is controlled to 7 to 10, and the iron sulfate dilution liquid is added dropwise at a rate of 200 mL/hr to 300 mL/hr, thereby coating the flake substrates.

When a rate at which the iron sulfate dilution liquid is added dropwise is less than 200 mL/hr, coating efficiency is increased, but blackness degree is decreased, and when a rate at which the iron sulfate dilution liquid is added dropwise is more than 300 mL/hr, coating efficiency is decreased, but blackness degree is increased.

In the coating of the flake substrates (S130), once the coating reaction of the aqueous inorganic salt is completed, the mixed solution containing the flake substrates is subjected to reflux for 30 minutes to 60 minutes. Then, the pH of the refluxed mixed solution is controlled to pH 7 to 10, followed by refluxing for 30 minutes to 60 minutes, thereby preparing a reaction solution. When the coating temperature is less than 80°C, thermal stability of the iron oxide layer to be coated may be reduced. When pH is low as being less than 7, the coating reaction may not be well performed, and when pH is excessively high as being higher than 10, an efficiency of the coating reaction may be largely reduced.

After the coating of the flake substrates (S130), the flake substrates coated with the oxide layer and a reaction residual liquid after the coating reaction is completed are washed with deionized water and dehydrated.

Then, the dehydrated flake substrates were dried at 50°C to 60°C. When the drying temperature is low as being less than 50°C, time required for the drying may be increased, and when the drying temperature is excessively high as being more than 60°C, color of the pigment may be changed due to the drying process for a long period of time at high temperature.

According to the present invention, the iron oxide-based black glossy pigment including Fe₃O₄ may be formed by using Fe²⁺ metal salt as a starting material rather than mixing Fe²⁺ with Fe³⁺ salt to form a pigment including Fe₃O₄ according to the related art.

In addition, since the method of the present invention has a relatively low reaction temperature and does not require an additional sintering process, as compared to a method for reducing a surface of Fe₂O₃ and a method for sintering Fe₂O₃ under reduction conditions, both of which are the existing method for preparing a soft magnetic iron oxide, stability of a reaction process may be secured, and the production cost may be reduced.

Hereinafter, constitution and function of the present invention will be described in more detail through preferably exemplary embodiments of the present invention. It is to be noted that Examples to be described below are provided merely for specifically exemplifying the present invention, and accordingly, the present invention is not limited to the following Examples.

Descriptions which are not described in the specification can be sufficiently and technically deduced by a person skilled in the technical field, and accordingly, details thereof will be omitted.

### Example

### [Preparation of coating liquid]

1194 g of deionized water and 30 g of sulfuric acid (98%) were mixed with each other to prepare a sulfuric acid dilution liquid, and 294 g of iron sulfate (FeSO₄·7H₂O) was added to the dilution liquid and mixed with each other. The mixed solution was refluxed for 30 minutes so as to dissolve the iron sulfate, thereby preparing a coating liquid.

A temperature of the primarily completed sample was controlled to 20°C to 30°C, and 74 g of potassium nitrate (KNO₃) was added to the sample, and the solid phase sample was dissolved for 30 minutes, thereby preparing a secondary mixed solution.

### [Coating Reaction]

1000 g of deionized water was added and dispersed into 100 g of the substrate, and a temperature of the reaction solution was raised to 75°C. Then, a coating reaction was performed at 75°C and pH 1.5 to 2.5 with 100 mL of Ti-metal salt dilution liquid. After the coating reaction was completed, the reaction solution was refluxed for 30 minutes, and the reaction conditions were controlled to 80°C and pH 9.0. Then, the reaction solution was refluxed for 30 minutes to stabilize the reaction solution.

The dilution liquid (coating liquid) prepared under the above-described condition was added at a rate of 250 mL/hr to thereby form a black iron oxide layer having magnetism. After the reaction was completed, the obtained sample was washed with deionized water and dehydrated. Then, a drying process was performed at 60°C or less for 12 hours or more.

### Comparative Example

### [Coating Reaction]

100 g of the substrate was added and dispersed into 1000 g of deionized water, and a temperature of the reaction solution was raised to 75°C. Then, the reaction solution was titrated with an appropriate amount of FeCl₃ dilution liquid at pH 3.0. After the coating reaction was completed, pH of the reaction solution was controlled to pH 9.0, and an appropriate amount of cobalt sulfate dilution liquid was coated thereonto to thereby form a black layer. After the formation of the black layer was completed, the reaction solution was washed and dehydrated. The dehydrated sample was dried and sintered at about 800°C to obtain a sample.

### Measurement of blackness degree

Color difference value of the iron oxide-based black pigment prepared by Example of the present invention was measured and compared with the color difference value of Comparative Example. Comparison results thereof were shown in Table 1 below.

The measurement was performed by a color difference meter (measured by MINOLTA cm-512m³ at 75 degrees) on a black background after drawdown of the samples to PC 6% (with NC Resin) on an opacity chart.

**[Table 1]**

| | Existing black pigment | Iron oxide-based black pigment |
|---|---|---|
| L | 53.35 | 23.32 |
| a | 0.91 | 0.79 |
| b | 2.94 | -0.49 |

As the measurement results of the color difference value, the iron oxide-based black pigment of Example had a lower gloss and lower absolute values of a and b as compared to the black pigment of Comparative Example. It could be confirmed from the result that the iron oxide-based black pigment of Example had a black color which was closer to achromatic color as compared to the black pigment of Comparative Example.

### Measurement of saturation magnetization

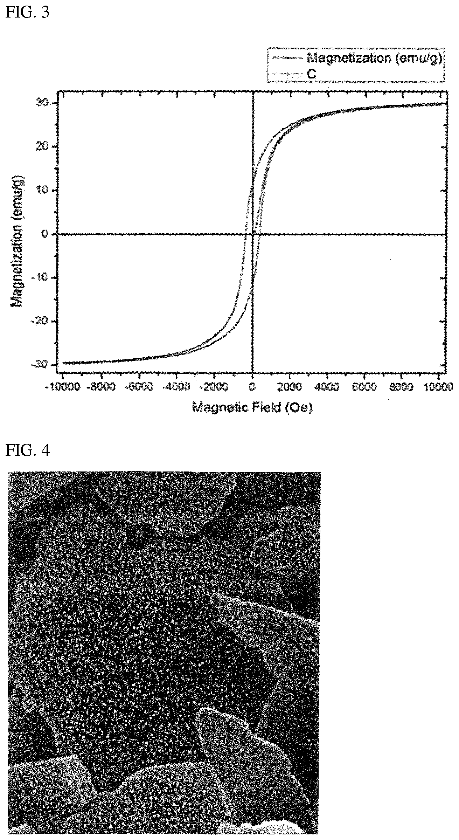

In order to confirm magnetic properties of the iron oxide-based black glossy pigments of Example and the black pigment of Comparative Example of the present invention, saturation magnetization values were measured by using a vibrating sample magnetometer (VSM), and results thereof were shown in FIGS. 2 and 3, respectively.

Referring to FIGS. 2 and 3, the black pigment prepared by Comparative Example had a saturation magnetization of about 18 emu/g (see FIG. 2); on the contrary, the iron oxide-based black glossy pigment prepared by Example had a saturation magnetization of about 30 emu/g (see FIG. 3). It could be appreciated from the results that the iron oxide-based black glossy pigment of Example had more increased saturation magnetization force than that of the black pigment of Comparative Example.

### Application Examples

The iron oxide-based black glossy pigment prepared according to Example of the present invention could be utilized as pigments of cosmetic products such as a mascara, an eye shadow, an eye liner (liquid and gel), a nail enamel, etc.

Tables 2 to 6 show compositions of mascara, eye shadow, liquid eye liner, gel eye liner, and nail enamel including the pigment of Example of the present invention.

**[Table 2]**

| Raw material | Amount |
|---|---|
| Cetearyl alcohol | 2.00 |
| PEG20 glyceryl stearate | 1.50 |
| Beeswax | 11.00 |
| Stearic acid | 8.00 |
| Black iron oxide | 3.00 |
| Preservative | 0.50 |
| Black pigment | 7.00 |
| Butylene glycol | 2.00 |
| Acrylate copolymer | 30.00 |
| Polyvinyl alcohol | 3.00 |
| Triethanolamine | 3.00 |
| Deionized water (D.I.water) | 29.00 |
| Sum | 100.00 |

Table 2 above shows composition of a mascara to which the pigment of Example of the present invention is applied.

**[Table 3]**

| Raw material | Amount |
|---|---|
| Black pigment | 10.0 |
| Talc | 68.40 |
| Mica | 7.20 |
| Zinc stearate | 5.40 |
| Silica | 4.50 |
| Methyl methacrylate copolymer | 2.88 |
| Titanium dioxide | 1.44 |
| Aluminum myristate | 0.09 |
| Triethoxycaprylylsilane | 0.05 |
| Dimethicone | 0.04 |
| Sum | 100.00 |

Table 3 above shows composition of an eye shadow to which the pigment of Example of the present invention is applied.

**[Table 4]**

| Raw material | Amount |
|---|---|
| Deionized water (D.I. water) | 32.87 |
| Butylene glycol | 1.00 |
| Sodium polyacrylate | 1.00 |
| Disodium EDTA | 0.03 |
| Black iron oxide | 10.00 |
| Black pigment | 14.00 |
| Silica | 0.50 |
| Preservative | 0.30 |
| Silicone oil | 2.00 |
| Caprylic / Capric triglyceride | 2.00 |
| Di-isostearyl malate | 2.00 |
| Cetearyl olivate / Sorbitan olivate | 2.00 |
| Polyoxyethylene lauryl ether | 2.00 |
| Preservative | 0.30 |
| Acrylate copolymer | 30.00 |
| Sum | 100.00 |

Table 4 above shows composition of a liquid eye liner to which the pigment of Example of the present invention is applied.

**[Table 5]**

| Raw material | Amount |
|---|---|
| Ceresin | 18.00 |
| Sodium polyacrylate | 3.00 |
| Silicone acrylate | 2.00 |
| Cyclomethicone | 10.70 |
| Silicone oil | 3.00 |
| Preservative | 0.30 |
| Isododecane | 10.00 |
| Caprylic / Capric triglyceride / Stearalkonium Hectorite / Propylene carbonate | 33.00 |
| Black pigment | 20.00 |
| Sum | 100.00 |

Table 5 above shows composition of a gel eye liner to which the pigment of Example of the present invention is applied.

**[Table 6]**

| Raw material | Amount |
|---|---|
| Black pigment | 3.00 |
| Nitrocellulose (1/2 second) | 10.00 |
| Alkyd resin | 10.00 |
| Citric acid acetyl tributyl | 2.00 |
| Ethyl acetate | 20.00 |
| Butyl acetate | 15.00 |
| Ethyl alcohol | 5.00 |
| Toluene | 35.00 |
| Sum | 100.00 |

Table 6 above shows composition of a nail enamel to which the pigment of Example of the present invention is applied.

Properties of spreadability, coverage, color, matt, etc., of the mascara, the eye shadow, the liquid eye liner, the gel eye liner, and nail enamel prepared by the compositions shown in Tables 2 to 6 were shown in Table 7 below.

**[Table 7]**

| Cosmetic type | Spreadability | Coverage | Color | Matt |
|---|---|---|---|---|
| Mascara | ⊙ | ⊙ | ⊚ | ⊙ |
| Eye Shadow | ⊚ | ⊙ | ⊚ | ⊚ |
| Eyeliner (liquid) | ⊙ | ⊚ | ⊙ | ⊚ |
| Eyeliner(gel) | ⊙ | ⊙ | ⊚ | ⊙ |
| Nail enamel | ⊙ | ⊙ | ⊚ | ⊚ |

| | | | | |
|---|---|---|---|---|
| × (less than 60 points) : Bad Δ (60 to 69 points) : Medium ○ (70 to 79 points) : Relatively Good ⊚ (80 to 89 points) : Excellent ⊙ (90 to 100 points) : Significantly Excellent | | | | |

Table 7 above shows evaluation results of spreadability, coverage, color and matt of each product, wherein the evaluation results were obtained by performing sensory evaluation (0 to 100 points) of usability, etc., as cosmetics at the time of using each product targeting women on the panel in their 20's up to 40's and scoring, then evaluating sections in which average values thereof are included.

Referring to Table 7 above, it could be confirmed that when the pigment of Example of the present invention is used as the cosmetic pigment, spreadability, coverage, clarity, and excellent color were shown. It indicated that the iron oxide-based black glossy pigment according to the present invention could express color by low gloss, while simultaneously maintaining functions that are equal or greater than those of generally used cosmetic pigments.

FIG. 4 is a scanning electron microscope (SEM) image of a surface of the iron oxide-based black pigment glossy pigment prepared according to Example of the present invention.

It could be appreciated from FIG. 4 that the iron oxide-based black pigment of the present invention was uniformly coated on a base material.

According to the present invention, the iron oxide-based black glossy pigment including Fe₃O₄ may be formed by using Fe²⁺ metal salt as a starting material rather than mixing Fe²⁺ with Fe³⁺ salt to form a pigment including Fe₃O₄ according to the related art.

In addition, since the method of the present invention has a relatively low reaction temperature and does not require an additional sintering process, as compared to a method for reducing a surface of Fe₂O₃ and a method for sintering Fe₂O₃ under reduction conditions, both of which are the existing methods for preparing a soft magnetic iron oxide, stability of a reaction process may be secured, and the production cost may be reduced.

Reference signs are incorporated in the claims solely to ease their understanding, and do not limit the scope of the claims.

## Claims

1. A method for preparing an Fe₃O₄ iron oxide-based black glossy pigment comprising:
preparing an iron sulfate dilution liquid (S110) containing iron sulfate (FeSO₄·7H₂O);
forming a substrate suspension by adding flake substrates (S120) to deionized water (D.I. water), followed by mixing, stirring and dispersing;
coating the flake substrates (S130) by titrating the substrate suspension with an aqueous inorganic salt solution to hydrolyze the aqueous inorganic salt solution, and mixing the prepared iron sulfate dilution liquid therewith, to thereby coat an oxide layer on surfaces of the flake substrates (S130); and
after the coating of the flake substrates, dehydrating and washing the flake substrates coated with the oxide layer and a reaction residual liquid, and drying the dehydrated flake substrates at 50°C to 60°C, and
screening the flake substrates, using mesh
wherein the aqueous inorganic salt solution includes one selected from TiCl₄, TiOCl₂, TiOSO₄, BaCl₂, AlCl₃, SnCl₄, FeCl₃, FeSO₄, SiCl₄, ZrOCl₂, Na₂O·SiO₂·5H₂O, MnCl₂, MgCl₂ and CoCl₂, or one or more mixtures thereof, and
wherein in the coating of the flake substrates (S130), at pH of 7 to 10, the substrate suspension is stirred and heated at 80°C to 85°C at 400 rpm to 450 rpm, and the iron sulfate dilution liquid is added dropwise to the substrate suspension at a rate of 200 mL/hr to 300 mL/hr.

2. The method of claim 1, wherein the preparing of the iron sulfate dilution liquid (S110) containing iron sulfate (FeSO₄·7H₂O) includes:
preparing a sulfuric acid dilution liquid by mixing 100 parts by weight of water with 1 to 6 parts by weight of sulfuric acid;
preparing a first mixed solution containing iron sulfate and acid by mixing 100 parts by weight of the prepared sulfuric acid dilution liquid with 10 to 40 parts by weight of iron sulfate (FeSO₄·7H₂O); and
preparing a second mixed solution by mixing and stirring 100 parts by weight of the first mixed solution with 3 to 10 parts by weight of at least one of KNO₃, NaNO₃, Ca(NO₃)₂ and Mg(NO₃)₂.

3. The method of claim 1 or 2, wherein the flake substrate includes at least one of synthetic mica, natural mica, glass, plate-shaped iron oxide, plate-shaped alumina and plate-shaped silica, talc, lead carbonate, and plate-shaped bismuth oxychloride (BiOCl).

4. The method of any previous claim, wherein in the forming of the substrate suspension, 100 parts by weight of the flake substrates are mixed with 900 to 1200 parts by weight of D.I. water, followed by heating and stirring at 70°C to 80°C at 400 rpm to 450 rpm,
in the coating of the flake substrates, at pH of 1.5 to 2.5, the substrate suspension is stirred and heated at 70°C to 80°C at 400 rpm to 450 rpm, and 8 to 12 parts by weight of the aqueous inorganic salt solution is added dropwise to 100 parts by weight of the substrate suspension, and
the coating of the flake substrates includes, after coating the mixed solution containing the flake substrates with inorganic salts, refluxing for 30 minutes to 60 minutes, controlling the refluxed mixed solution to pH 7 to 10, and refluxing the mixed solution for 30 minutes to 60 minutes.

## Patentansprüche

1. Verfahren zum Herstellen eines schwarzen Hochglanzpigments auf Fe₃O₄ Eisenoxidbasis, das Folgendes umfasst:
Herstellen einer Eisensulfatverdünnungsflüssigkeit (S110), die Eisensulfat (FeSO₄·7H₂O) enthält;
Ausbilden einer Substratsuspension durch Zugeben von Flockensubstraten (S120) zu entionisiertem Wasser (*deionized water-* DI-Wasser), gefolgt von Mischen, Rühren und Dispergieren;
Beschichten der Flockensubstrate (S130) durch Titrieren der Substratsuspension mit einer wässrigen anorganischen Salzlösung, um die wässrige anorganische Salzlösung zu hydrolysieren, und Mischen der hergestellten Eisensulfatverdünnungsflüssigkeit damit, um dadurch eine Oxidschicht auf Oberflächen der Flockensubstrate (S130) zu beschichten; und
nach dem Beschichten der Flockensubstrate, Dehydratisieren und Waschen der mit der Oxidschicht und einer Reaktionsrestflüssigkeit beschichteten Flockensubstrate und Trocknen der dehydratisierten Flockensubstrate bei 50 °C bis 60 °C und
Sieben der Flockensubstrate unter Verwendung eines Netzes,
wobei die wässrige anorganische Salzlösung eine beinhaltet, die aus TiCl₄, TiOCl₂, TiOSO₄, BaCl₂, AlCl₃, SnCl₄, FeCl₃, FeSO₄, SiCl₄, ZrOCl₂, Na₂O·SiO₂·5H₂O, MnCl₂, MgCl₂ und CoCl₂ oder einer oder mehreren Mischungen davon ausgewählt ist, und wobei bei dem Beschichten der Flockensubstrate (S130), bei einem pH-Wert von 7 bis 10, die Substratsuspension auf 80 °C bis 85 °C bei 400 rpm bis 450 rpm gerührt und erhitzt wird, und die Eisensulfatverdünnungsflüssigkeit mit einer Geschwindigkeit von 200 ml/h bis 300 ml/h zu der Substratsuspension tropfenweise zugegeben wird.

2. Verfahren nach Anspruch 1, wobei das Herstellen der Eisensulfatverdünnungsflüssigkeit (S110), die Eisensulfat (FeSO₄·7H₂O) enthält, Folgendes beinhaltet:
Herstellen einer Schwefelsäureverdünnungsflüssigkeit durch Mischen von 100 Gewichtsteilen Wasser mit 1 bis 6 Gewichtsteilen Schwefelsäure;
Herstellen einer ersten gemischten Lösung, die Eisensulfat und Säure enthält, durch Mischen von 100 Gewichtsteilen der hergestellten Schwefelsäureverdünnungsflüssigkeit mit 10 bis 40 Gewichtsteilen Eisensulfat (FeSO₄·7H₂O); und
Herstellen einer zweiten gemischten Lösung durch Mischen und Rühren von 100 Gewichtsteilen der ersten gemischten Lösung mit 3 bis 10 Gewichtsteilen von wenigstens einem von KNO₃, NaNO₃, Ca(NO₃)₂ and Mg(NO₃)₂.

3. Verfahren nach Anspruch 1 oder 2, wobei das Flockensubstrat wenigstens eines von synthetischem Glimmer, natürlichem Glimmer, Glas, plattenförmigem Eisenoxid, plattenförmigem Aluminiumoxid und plattenförmigem Siliciumdioxid, Talk, Bleicarbonat und plattenförmigem Wismutoxychlorid (BiOCI) beinhaltet.

4. Verfahren nach einem vorherigen Anspruch, wobei bei dem Ausbilden der Substratsuspension 100 Gewichtsteile der Flockensubstrate mit 900 bis 1200 Gewichtsteilen DI-Wasser gemischt werden, gefolgt von einem Erhitzen und einem Rühren auf 70 °C bis 80 °C bei 400 rpm bis 450 rpm,
bei dem Beschichten der Flockensubstrate, bei dem pH-Wert von 1,5 bis 2,5, die Substratsuspension auf 70 °C bis 80 °C bei 400 rpm bis 450 rpm gerührt und erhitzt wird, und 8 bis 12 Gewichtsteile der wässrigen anorganischen Salzlösung zu 100 Gewichtsteilen der Substratsuspension tropfenweise zugegeben werden, und
das Beschichten der Flockensubstrate, nach dem Beschichten der gemischten Lösung, die die Flockensubstrate mit anorganischen Salzen enthält, ein Sieden unter Rückfluss 30 Minuten bis 60 Minuten lang, ein Steuern der unter Rückfluss gesiedeten gemischten Lösung auf den pH-Wert 7 bis 10 und das Sieden unter Rückfluss der gemischten Lösung 30 Minuten bis 60 Minuten lang beinhaltet.

## Revendications

1. Procédé de préparation d'un pigment noir brillant à base d'oxyde de fer Fe₃O₄ comprenant :
la préparation d'un liquide de dilution de sulfate de fer (S110) contenant du sulfate de fer (FeSO₄·7H₂O) ;
le formage d'une suspension de substrat en ajoutant des substrats en paillettes (S120) à de l'eau désionisée (eau D.I.), puis en mélangeant, en agitant et en dispersant ;
le revêtement des substrats en paillettes (S130) par titrage de la suspension de substrat avec une solution aqueuse de sel inorganique pour hydrolyser la solution aqueuse de sel inorganique, et le mélange du liquide de dilution de sulfate de fer préparé avec celle-ci, pour ainsi revêtir une couche d'oxyde sur les surfaces des substrats en paillettes (S130) ; et
après le revêtement des substrats en paillettes, la déshydratation et le lavage des substrats en paillettes revêtus de la couche d'oxyde et d'un liquide résiduel de réaction, et le séchage des substrats en paillettes déshydratés entre 50 °C et 60 °C, et
le tamisage des substrats en paillettes, à l'aide d'une maille,
une solution aqueuse de sel inorganique comportant un composé choisi parmi du TiCl₄, TiOCl₂, TiOSO₄, BaCl₂, AlCl₃, SnCl₄, FeCl₃, FeSO₄, SiCl₄, ZrOCl₂, Na₂O·SiO₂·5H₂O, MnCl₂, MgCl₂ et CoCl₂, ou parmi un ou plusieurs mélanges de ceux-ci, et
lors du revêtement des substrats en paillettes (S130), à un pH de 7 à 10, la suspension de substrat étant agitée et chauffée de 80 °C à 85 °C de 400tr/min à 450 tr/min, et le liquide de dilution de sulfate de fer étant ajouté goutte à goutte à la suspension de substrat à une vitesse de 200 ml/h à 300 ml/h.

2. Procédé selon la revendication 1, dans lequel la préparation du liquide de dilution de sulfate de fer (S110) contenant du sulfate de fer (FeSO₄·7H₂O) comporte :
la préparation d'un liquide de dilution d'acide sulfurique en mélangeant 100 parties en poids d'eau avec 1 à 6 parties en poids d'acide sulfurique ;
la préparation d'une première solution mélangée contenant du sulfate de fer et de l'acide en mélangeant 100 parties en poids du liquide de dilution d'acide sulfurique préparé avec 10 à 40 parties en poids de sulfate de fer (FeSO₄·7H₂O) ; et
la préparation d'une seconde solution mélangée en mélangeant et en agitant 100 parties en poids de la première solution mélangée avec 3 à 10 parties en poids d'au moins l'un parmi le KNO₃, le NaNO₃, le Ca(NO₃)₂ et le Mg(NO₃)₂.

3. Procédé selon la revendication 1 ou 2, dans lequel le substrat en paillettes comporte au moins l'un parmi le mica synthétique, le mica naturel, le verre, l'oxyde de fer en forme de plaque, l'alumine en forme de plaque et la silice en forme de plaque, le talc, le carbonate de plomb, et l'oxychlorure de bismuth en forme de plaque (BiOCl).

4. Procédé selon l'une quelconque des revendications précédentes, dans lequel, lors du formage de la suspension de substrat, 100 parties en poids des substrats en paillettes sont mélangées avec 900 à 1 200 parties en poids d'eau D.I., puis chauffées et agitées de 70 °C à 80 °C de 400 tr/min à 450 tr/min,
au cours du revêtement des substrats en paillettes, à un pH de 1,5 à 2,5, la suspension de substrat est agitée et chauffée de 70 °C à 80 °C de 400 tr/min à 450 tr/min, et 8 à 12 parties en poids de la solution aqueuse de sel inorganique sont ajoutées goutte à goutte à 100 parties en poids de la suspension de substrat, et
le revêtement des substrats en paillettes comporte, après le revêtement de la solution mélangée contenant les substrats en paillettes avec des sels inorganiques, le reflux pendant 30 minutes à 60 minutes, la régulation de la solution mélangée refluée à un pH 7 à 10 et le reflux de la solution mélangée pendant 30 minutes à 60 minutes.
